# EUROPEAN PATENT SPECIFICATION

(11) **EP 2 722 204 B1**
(45) Date of publication and mention of the grant of the patent: **04.11.2020**
(21) Application number: 13004534.7
(22) Date of filing: 17.09.2013
(51) Int. Cl.: B60G 17/017, B60T 7/16

(54) **Portable electronic device for changing trailer height using the pneumatic suspension**
Tragbares elektronisches Gerät zum Ändern der Anhängerhöhe mit der Luftfederung
Dispositif électronique portable pour modifier la hauteur de la remorque à l'aide de la suspension pneumatique

(30) Priority: 17.09.2012 GB 201216564
(43) Date of publication of application: 23.04.2014
(73) Proprietor: Knorr-Bremse Systems for Commercial Vehicles Limited, Bristol BS16 7FE (GB); KNORR-BREMSE Systeme für Nutzfahrzeuge GmbH, 80809 München (DE)
(72) Inventor: Fry, Matthew, Bristol, BS16 7FE (GB); Lumley, Andrew, Bristol, BS16 7FE (GB); Pahl, Stefan, D-80809 Munchen (DE); Mederer, Martin, D-80809 Munchen (DE)
(74) Representative: JENSEN & SON

(56) References cited:
- EP-A2- 1 282 298
- GB-A- 2 419 718
- US-A1- 2004 070 160

## Description

The invention relates to a trailer electronic braking system.

Vehicle trailers such as those used in the distribution of goods are provided with an air suspension system, in which the suspension is provided by airbags at each axle end. The pressure in the airbags is adjustable depending on loading conditions on the trailer.

As loading bays used in distribution depots are not of a standard height, a raise/lower valve is provided so that the height of the trailer can be changed from the drive position, which is the ideal height for driving conditions, by increasing or reducing the amount of air in the airbags. Raise/lower valves arc typically manually controlled using a lever or buttons but it is known to provide the suspension system with a so-called reset to ride valve, which ensures that the trailer is automatically set to the correct height for driving conditions in the event that the trailer is driven in a raised or lowered position.

The modification of trailer height in the known systems operates in the presence of the operator. The modification of the air suspension to change the trailer height if the operator were not present is potentially hazardous as the trailer height could be changed during a loading or unloading operation.

DE102008028434 discloses a loading platform for use on a transport vehicle. The platform comprises a control wirelessly connected to an operating unit. Control commands inputted into the operating unit are transmitted to the control by the wireless connection. The control and/or operating unit comprises a request unit which sets safety criteria.

US2004/0070160 discloses an automatic trailer suspension control system arranged to regulate vehicle height during loading and/or unloading. The trailer suspension control system is connected to a standard braking system and comprises a control valve operable by an electronic control unit (ECU) provided with a standards compliant communication interface. The ECU is adapted to communicate wirelessly with a transmission device.

The present invention seeks to provide vehicle function control apparatus that is remotely or wirelessly operable.

According to the invention there is provided a trailer vehicle function control system for a trailer, the system comprising an electronic control unit, which electronic control unit is provided with a standards compliant communications interface adapted to communicate wirelessly with an electronic device remote from the communications interface adapted to actuate control of functions on the trailer, characterised in that the electronic control unit is adapted in a first step to transmit a signal at a first strength and the electronic device is adapted to measure the signal strength of signals received from the electronic control device and to return the measured signal strength, wherein the electronic control unit compares the transmitted signal strength with the received signal strength returned by the electronic device, the electronic control unit then determines a value for the proximity of the electronic device based on the difference between the transmitted and received signal strengths.

Preferably, the electronic control unit transmits initially at maximum strength.

Preferably, if the difference between the transmitted and received signal strengths exceeds a threshold value, control of vehicle functions from the electronic device is disabled.

Preferably, the electronic control unit measures the signal strength received from the electronic device and compares the difference between the transmitted signal strength with the received signal strength at the electronic control unit and further compares this difference with the difference between the transmitted signal strength and the signal strength received at the electronic device.

Preferably, the electronic control unit sends a message to the electronic device if the measured signal strength is below a predetermined threshold. Preferably, the control function controls the air suspension so that the height of the trailer can be raised or lowered by changing the air pressure in air bags associated with an axle of the trailer.

Preferably, the electronic control unit is the trailer braking electronic control unit. Preferably, the communication interface comprises a 802.11 wireless card, which card is adapted to transmit an SSID, the trailer identifier comprising the SSID. Preferably, the electronic control unit is provided with a list of MAC addresses associated with allowable portable electronic devices, the control unit detecting the MAC address of any further devices it detects such that it only pairs with an allowable MAC address.

Exemplary embodiments of the invention will now be described in greater detail with reference to the drawings in which:

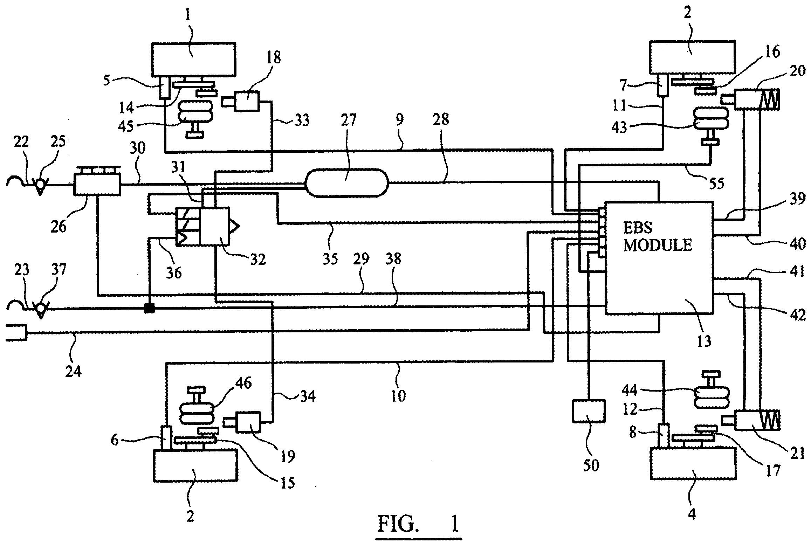

Fig. 1 shows a trailer electronic braking system
Fig. 2 shows a schematic diagram of the brake control with a trailer access microcontroller

The utility vehicle trailer has a steerable front axle with front wheels 1, 2 and a rear axle with rear wheels 3, 4. Rotational wheel speed sensors 5-8 are in each case assigned to the front wheels 1, 2 and the rear wheels 3, 4, and are connected by way of electric lines 9-12 with an electropneumatic brake pressure control module 13 (EBS module) which is primarily assigned to the rear axle brakes. One brake 14-17 is in each case assigned to the front wheels 1, 2 and the rear wheels 3, 4, which brake 14-17 can be applied by means of brake cylinders 18, 19 of the front axle or spring-loaded brake cylinders 20, 21 of the rear axle.

The braking system of the trailer vehicle can be connected by way of three connections, specifically a pneumatic supply line connection 22, a pneumatic control line connection 23 and an electric control connection 24, with the braking system of a tractor or a further trailer. The electric control line 24 provides the ISO 11992 CAN data connection.

The supply line connection 22 is connected by way of a filter valve 25 and a parking valve 26 with an air brake reservoir 27. From the air brake reservoir 27, a pneumatic line 28, 30 leads to a supply input of the pressure control module 13 and electropneumatic valve 32, which is adapted to supply ABS functionality. In addition, a pneumatic line 29 branches off the parking valve 26 to the pressure control module 13. A pneumatic line 30 extends between the parking valve 26 and the air brake reservoir 27.

The electropneumatic valve 32 is assigned jointly to both brake cylinders 18, 19 of the front axle and is connected with the brake cylinder 18 by way of a pneumatic line 33 and with the brake cylinder 19 by way of a pneumatic line 34. The valve 32 has two electric control inputs which are connected by way of "one" electric communication line 35 such as CAN, shown here only schematically, with the pressure control module 13.

Furthermore, the valve 32 has a pneumatic control input 36 which is connected by way of a filter valve 37 with the pneumatic control connection 23. The pneumatic control input 36 is also connected by way of a pneumatic control line 38 with a pneumatic control input of the pressure control module 13. The pressure control module 13 has an integrated pressure sensor (not shown) which measures the pressure in the pneumatic control line 38, that is, the control pressure present at the pneumatic control input 36 of the electropneumatic valve, which control pressure is identical to the maximal pressure which can be controlled into the brake cylinders 18, 19.

The pressure control module 13 has pneumatic outputs 39-42 which are connected by way of assigned pneumatic lines with the spring brake cylinders 20 or 21.

Furthermore, air bags 43, 44 are provided at the rear axle and permit a determination of the axle load, particularly of the dynamic axle load during braking and starting. The air bags 43, 44 are connected by way of pneumatic lines with the pressure control module 13. The pressure control module 13 has an integrated pressure sensor (not shown) which measures the pressure in the air bags 43, 44. Correspondingly the pressure in airbags 45, 46 provided at the front axle, which here are electrically controlled, may be detected by an optional pressure transducer. The pressure in the airbags 43-46 can be increased or decreased so as to adjust the height of the trailer.

To provide stability control a lateral acceleration sensor is provided, which may also be integrated with a yaw sensor, and the output of the lateral acceleration sensor is fed to the pressure control module/ECU 13. Typically the lateral acceleration sensor is integrated into the pressure control module/ECU 13. In the event that lateral acceleration on the trailer is detected, the pressure control module can provide for increased brake force at the front and/or rear axles. When the lateral acceleration sensor detects lateral acceleration on the trailer in which it is installed, the sensor generates a signal setting the stability control to active.

The pressure control module 13 receives data from the wheel speed sensors on the trailer and also receives a signal indicating whether the brake pedal in the vehicle cab is depressed or not, as well as the brake pressure demand.

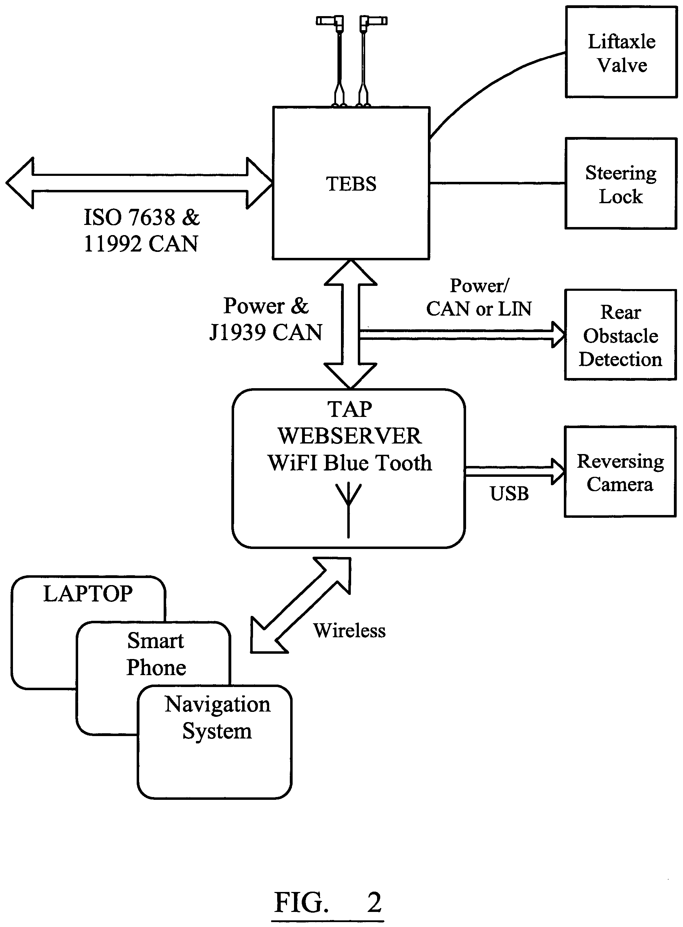

Figure 2 shows schematically the arrangement of the trailer access point microcontroller with the trailer electronic braking system. The trailer electronic braking system is only partially illustrated for reasons of clarity but includes the pressure control module 13 which is shown receiving wheel speed signals from wheel speed sensors 5,6. The pressure control module 13 also receives inputs from the lift axles showing the position of the lift axles and also the steering lock. The pressure control module 13 also receives power and data via an electrical connection from the tractor and is also attached to an ISO 11992 CAN databus.

A trailer access point (TAP) comprising microcontroller with a CAN interface is also provided, which is powered from the electrical connection. The microcontroller itself comprises an interface to the on-board electronic systems and the trailer electronic braking system and an interface for sending and receiving data in a wireless format. The communications interface can comprise one or more of a 802.11 transceiver or Bluetooth transceiver or radio transceiver. 802.11 and Bluetooth signals can have a reception and transmission range of up to 100 metres in free air. The trailer access point is connected to the CAN bus on the trailer and so is able to receive data from other devices on the CAN bus. The trailer access point can further be provided with USB ports, which enables the addition of peripheral devices on the trailer. An exemplary device on the CAN bus is the rear obstacle detector and an exemplary device attached to the USB port is a reversing camera. USB is preferable in this case as the camera would generate large amounts of data compared to the remaining data on the CAN bus.

The trailer access point can be mounted in a housing similar to that used for a trailer information module but without a display being necessary. If a display is needed, it would be possible to use a bistable cholestatic display or zero power LCD display.

In use, when the trailer access point is powered, the provision of the access point enables the data from the devices to be read by another device with a client browser. Such devices could include a tractor navigation system or a smartphone. It would also be possible to use a laptop.

Each trailer is provided with a vehicle identification number (VIN), which is usually provided on a plate on the side of the trailer. These plates are also often provided with a barcode or other machine readable data recording the same information. The wireless communication card on the trailer access microcontroller is provided with its own identifier such as the service set identifier or SSID for an 802.11 card. The SSID can also preferably be set to be the same as or include the trailer VIN, The depot operator or truck driver is then provided with a device such as a smartphone so that the handheld device can be used to pair with the microcontroller and hence trailer with the truck. If the truck is provided with a navigation system, it would be possible to use this. To provide some additional security, the trailer access microcontroller may be provided with a list of allowed MAC addresses with which it is permitted to pair. As an alternative to using the SSID as an identifier, it would also be possible to use the fleet number as an identifier.

If the trailer access point is set to receive vehicle control functions via a wireless connection such as 802.11 or Bluetooth, it is possible to reduce the transmission power of the wireless signal to reduce power consumption on the device. This has the potential disadvantage in that it is known that portable electronic devices such as mobile phones adjust their own transmission power in inverse proportion to the received signal strength to ensure the communication remains as stable as possible.

The trailer access point can co-operate with the wireless control device. The trailer access point transmits at least initially using maximum transmission signal strength. The control device is capable of measuring the received signal strength and can be adapted to return the value of this measured signal strength to the trailer access point. The trailer access point can then use this information to determine approximately the proximity of the control device.

When the control device is receiving a signal which is good strength, it is likely to reduce its own transmission signal strength. This in turn is advantageous as the received signal strength can be used to augment the proximity decision as if both the received transmission signal echoed back from the control device and the actual received strength are both high then the wireless control device and the trailer access point are substantially co-located. The method can also be used when using web pages served by the trailer access point, where the web pages use embedded scripts to request the received signal strength from the control device.

It would be possible for the trailer access point to send a message to a portable electronic device being used for control functions advising that the signal strength is too low and where appropriate that the user should change their position to improve the signal strength.

Although the trailer access point has been specifically described as a microcontroller, it would be possible to incorporate the functionality as an auxiliary function in the trailer electronic brake control unit. The trailer electronic brake control unit receives the operational data from the sensor outputs but would need to have an additional communication interface to be able to provide the interface to the standards compliant browser or client.

## Claims

1. A trailer vehicle function control system for a trailer, the system comprising an electronic control unit, which electronic control unit is provided with a standards compliant communications interface adapted to communicate wirelessly with an electronic device remote from the communications interface adapted to actuate control of functions on the trailer,
**characterised in that** the electronic control unit is adapted in a first step to transmit a signal at a first strength and the electronic device is adapted to measure the signal strength of signals received from the electronic control device and to return the measured signal strength, wherein the electronic control unit compares the transmitted signal strength with the received signal strength returned by the electronic device, the electronic control unit then determines a value for the proximity of the electronic device based on the difference between the transmitted and received signal strengths.

2. A trailer vehicle function control system according to Claim 1, wherein the electronic control unit transmits initially at maximum strength.

3. A trailer vehicle function control system according to Claim 1 or Claim 2, wherein if the difference between the transmitted and received signal strengths exceeds a threshold value, control of vehicle functions from the electronic device is disabled.

4. A trailer vehicle function control system according to any one of Claims 1 to 3, wherein the electronic control unit measures the signal strength received from the electronic device and compares the difference between the transmitted signal strength with the received signal strength at the electronic control unit and further compares this difference with the difference between the transmitted signal strength and the signal strength received at the electronic device.

5. A trailer vehicle function control system according to any one of Claims 1 to 4, wherein the electronic control unit is the trailer braking electronic control unit.

6. A trailer vehicle function control system according to any one of Claims 1 to 5, wherein the communication interface comprises a 802.11 wireless card, which card is adapted to transmit an SSID, the trailer identifier comprising the SSID.

7. A trailer vehicle function control system according to any one of Claims 1 to 6, wherein the electronic control unit is provided with a list of MAC addresses associated with allowable portable electronic devices, the control unit detecting the MAC address of any further devices it detects such that it only pairs with an allowable MAC address.

8. A trailer vehicle function control system according to any one of Claims 1 to 7, wherein the electronic control unit sends a message to the electronic device if the measured signal strength is below a predetermined threshold.

9. A trailer vehicle function control system according to any one of Claims 2 to 8, wherein the control function controls the air suspension so that the height of the trailer can be raised or lowered by changing the air pressure in air bags associated with an axle of the trailer.

## Patentansprüche

1. Anhängerfahrzeug-Funktionssteuersystem für einen Anhänger, umfassend eine elektronische Steuereinheit mit einer normgemäßem Kommunikationsschnittstelle, die zur drahtlosen Kommunikation mit einem von der Kommunikationsschnittstelle entfernt angeordneten Elektronikgerät ausgelegt ist, das zur Aktivierung der Steuerung von Funktionen am Anhänger ausgelegt ist.
**dadurch gekennzeichnet, dass** die elektronische Steuereinheit dazu ausgelegt ist, in einem ersten Schritt ein Signal mit einer ersten Stärke zu übertragen, und dass das Elektronikgerät dazu ausgelegt ist, die Signalstärke der von der elektronischen Steuereinheit empfangenen Signale zu messen und die gemessene Signalstärke zurück zu senden, wobei die elektronische Steuereinheit die übertragene Signalstärke mit der empfangenen, vom Elektronikgerät zurück gesendeten Signalstärke vergleicht und die elektronische Steuereinheit dann einen Wert für die Nähe des Elektronikgeräts auf der Basis des Unterschiedes zwischen der übertragenen und der empfangenen Signalstärke bestimmt.

2. Anhängerfahrzeug-Funktionssteuersystem nach Anspruch 1, wobei die elektronische Steuereinheit anfänglich mit Maximalstärke überträgt.

3. Anhängerfahrzeug-Funktionssteuersystem nach Anspruch 1 oder Anspruch 2, wobei die Steuerung der Fahrzeugfunktionen durch das Elektronikgerät deaktiviert wird, wenn der Unterschied zwischen der übertragenen und der empfangenen Signalstärke einen Schwellenwert überschreitet.

4. Anhängerfahrzeug-Funktionssteuersystem nach einem der Ansprüche 1 bis 3, wobei die elektronische Steuereinheit die vom Elektronikgerät empfangene Signalstärke misst und den Unterschied zwischen der übertragenen Signalstärke mit der beim Elektronikgerät empfangenen Signalstärke vergleicht und weiter den besagten Unterschied mit dem Unterschied zwischen der übertragenen Signalstärke und der beim Elektronikgerät empfangenen Signalstärke vergleicht.

5. Anhängerfahrzeug-Funktionssteuersystem nach einem der Ansprüche 1 bis 4, wobei die elektronische Steuereinheit die elektronische Steuereinheit für Anhängerbremsung ist.

6. Anhängerfahrzeug-Funktionssteuersystem nach einem der Ansprüche 1 bis 5, wobei die Kommunikationsschnittstelle eine Drahtloskarte 802.11 umfasst, die zur Übertragung einer SSID ausgelegt ist, wobei das Anhängerkennzeichen die SSID umfasst.

7. Anhängerfahrzeug-Funktionssteuersystem nach einem der Ansprüche 1 bis 6, wobei die elektronische Steuereinheit mit einer Liste von MAC-Adressen versehen ist, die mit zulässigen tragbaren Elektronikgeräten verbunden sind, wobei die Steuereinheit die MAC-Adresse von etwaigen weiteren Geräten so erfasst, dass sie nur mit einer zulässigen MAC-Adresse gepaart werden.

8. Anhängerfahrzeug-Funktionssteuersystem nach einem der Ansprüche 1 bis 7, wobei die elektronische Steuereinheit eine Meldung an das Elektronikgerät sendet, wenn die gemessene Signalstärke unter einem vorgegebenen Schwellenwert liegt.

9. Anhängerfahrzeug-Funktionssteuersystem nach einem der Ansprüche 2 bis 8, wobei die Steuerfunktion die Luftfederung so steuert, dass die Höhe des Anhängers durch Ändern des Luftdrucks in mit der Achse des Anhängers verbundenen Luftkissen erhöht oder verringert werden kann.

## Revendications

1. Système de commande de fonction de véhicule à remorque pour une remorque, le système comprenant un module de commande électronique, lequel module de commande électronique est pourvu d'une interface de communications conforme aux normes adaptée à communiquer sans fil avec un dispositif électronique à distance de l'interface de communications adapté à actionner la commande de fonctions sur la remorque,
**caractérisé en ce que** le module de commande électronique est adapté dans une première étape à transmettre un signal à une première intensité et le dispositif électronique est adapté à mesurer l'intensité de signal de signaux reçus du dispositif de commande électronique et à renvoyer l'intensité de signal mesurée, dans lequel le module de commande électronique compare l'intensité de signal transmis à l'intensité de signal reçu renvoyée par le dispositif électronique, le module de commande électronique détermine ensuite une valeur pour la proximité du dispositif électronique en fonction de la différence entre les intensités de signal transmis et reçu.

2. Système de commande de fonction de véhicule à remorque selon la revendication 1, dans lequel le module de commande électronique transmet initialement à l'intensité maximale.

3. Système de commande de fonction de véhicule à remorque selon la revendication 1 ou la revendication 2, dans lequel si la différence entre les intensités de signal transmis et reçu dépasse une valeur seuil, la commande de fonctions de véhicule depuis le dispositif électronique est désactivée.

4. Système de commande de fonction de véhicule à remorque selon l'une quelconque des revendications 1 à 3, dans lequel le module de commande électronique mesure l'intensité de signal reçue du dispositif électronique et compare la différence entre l'intensité de signal transmis et l'intensité de signal reçu au niveau du module de commande électronique, et compare en outre cette différence à la différence entre l'intensité de signal transmis et l'intensité de signal reçue au niveau du dispositif électronique.

5. Système de commande de fonction de véhicule à remorque selon l'une quelconque des revendications 1 à 4, dans lequel le module de commande électronique est le module de commande électronique de freinage de remorque.

6. Système de commande de fonction de véhicule à remorque selon l'une quelconque des revendications 1 à 5, dans lequel l'interface de communication comprend une carte sans fil 802.11, laquelle carte est adaptée à transmettre un identifiant SSID, l'identifiant de remorque comprenant l'identifiant SSID.

7. Système de commande de fonction de véhicule à remorque selon l'une quelconque des revendications 1 à 6, dans lequel le module de commande électronique est pourvu d'une liste d'adresses MAC associées à des dispositifs électroniques portables autorisables, le module de commande détectant l'adresse MAC de tout autre dispositif qu'il détecte de sorte qu'il ne s'apparie qu'avec une adresse MAC autorisable.

8. Système de commande de fonction de véhicule à remorque selon l'une quelconque des revendications 1 à 7, dans lequel le module de commande électronique envoie un message au dispositif électronique si l'intensité de signal mesurée est en dessous d'un seuil prédéterminé.

9. Système de commande de fonction de véhicule à remorque selon l'une quelconque des revendications 2 à 8, dans lequel la fonction de commande commande la suspension d'air de sorte que la hauteur de la remorque peut être élevée ou abaissée en changeant la pression d'air dans des coussins d'air associés à un essieu de la remorque.
